# EUROPEAN PATENT SPECIFICATION

(11) **EP 1 100 321 B1**
(45) Date of publication and mention of the grant of the patent: **11.02.2004**
(21) Application number: 00927968.8
(22) Date of filing: 10.05.2000
(51) Int. Cl.: A01K 29/00, A01K 1/00

(54) **AN UNMANNED VEHICLE TO BE USED IN A STABLE OR A MEADOW**
UNBEMANNTES FAHRZEUG ZUR ANWENDUNG IN EINEM STALL ODER IN EINER WEIDE
VEHICULE SANS CONDUCTEUR S'UTILISANT DANS UNE ECURIE OU UN PRE

(30) Priority: 25.05.1999 NL 1012137
(43) Date of publication of application: 23.05.2001
(73) Proprietor: Lely Enterprises AG, 6300 ZUG (CH)
(72) Inventor: VAN DEN BERG, Karel, NL-2971 BR Bleskensgraaf (NL)
(74) Representative: Corten, Maurice Jean F.M.
(86) International application number: PCT/NL2000/000308
(87) International publication number: WO 2000/070941

(56) References cited:
- WO-A-96/14735
- WO-A-97/31524
- WO-A-98/47351
- DE-A- 2 909 325
- FR-A- 2 586 223
- US-A- 5 474 085
- US-A- 5 832 868
- PATENT ABSTRACTS OF JAPAN vol. 16, no. 120 (E-1182), 26 March 1992 (1992-03-26) & JP 03 289310 A (TOSHIBA CORP.), 19 December 1991 (1991-12-19)

## Description

The invention relates to an unmanned vehicle to be used in a stable, such as e.g. a cowshed, or in a meadow according to the preamble of claim 1.

Such a vehicle is known from US-A-5,474,085.

It is an object of the invention to provide an alternative unmanned vehicle.

This object is in accordance with the invention by the features of the characterizing part of claim 1. With the aid of its detection means the unmanned vehicle is capable of identifying animals which are ill and/or display abnormal behaviour. By means of the disinfecting means for disinfecting at least a part of an animal can be disinfected. According to again another aspect of the invention, the disinfecting means are disposed on a telescopic carrier. The latter measure makes it possible to disinfect at places, which are difficult to reach.

According to an inventive feature, the unmanned vehicle comprises alarm means for alarming a supervisor when an animal is ill or displays abnormal behaviour. In this manner it is possible to react quickly and adequately when there is something wrong with an animal.

According to a further inventive feature, the detection means comprise an animal identification system provided with a transmitter and a receiver. By means of the animal identification system there is determined for example whether an animal is lying or standing longer than usually at a certain place. This may be an indication that the animal is ill. According to another inventive feature, the animal identification system comprises a radar as well as reflectors reacting on the radar, which are disposed on the animals. Each of these reflectors has a unique code, so that it is possible to determine per animal how the animal is moving. In this manner it is also possible to determine abnormal behaviour of the animals. According to another inventive feature, the detection means comprise a camera, preferably constituted by an infrared camera. By means of image analysis of the images of the animals recorded by the camera it is possible for example to determine whether an animal has mastitis or is injured or has to be inseminated. It is also possible to track the animals by means of the camera. For enabling a still better view of the animals, the detection means are disposed on a telescopic carrier. According to again another inventive feature, the unmanned vehicle comprises driving means for driving the animals. With the aid of the driving means animals can be separated from a group, e.g. for the purpose of being inseminated or examined by a veterinary surgeon. In a preferred embodiment of the invention, the driving means comprise an electric shock device.

For the purpose of rendering the unmanned vehicle still more multifunctional, it is provided with a manure slide for removing manure which is lying on a floor. According to another inventive feature, the unmanned vehicle is provided with navigation means for guiding the unmanned vehicle through the stable or the meadow. The navigation means may be the same as the above-described detection means.

The invention will now be explained in further detail with reference to the accompanying drawings.

Figure 1 is a plan view of a stable with an unmanned vehicle accommodated therein, which vehicle is provided with detection means according to the invention, and

Figure 2 is a side view of the unmanned vehicle shown in Figure 1.

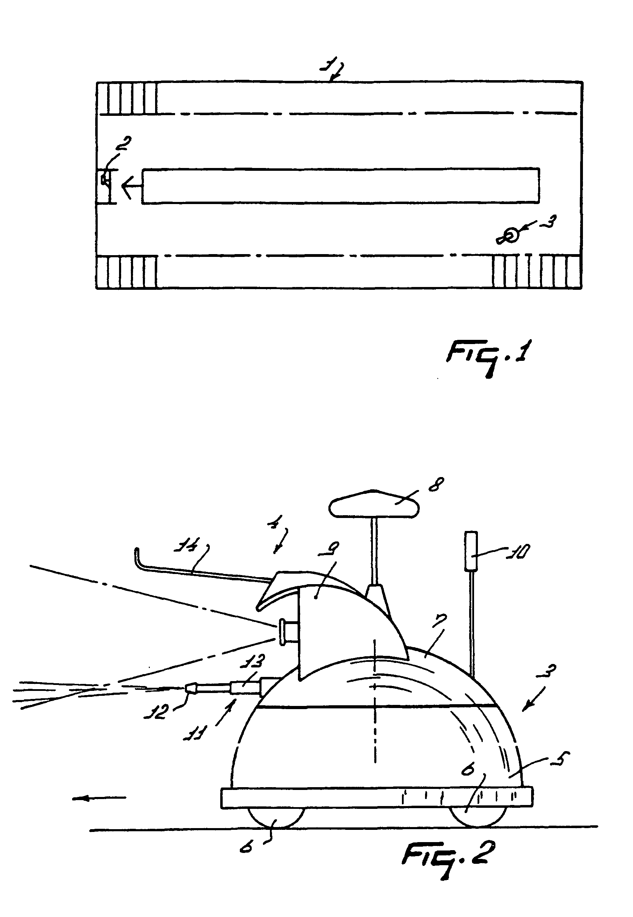

Figure 1 is a plan view of a stable 1 provided with a milking robot 2 for automatically milking animals and an unmanned vehicle 3 which is provided with detection means 4 for determining the health and/or the behaviour of animals.

Figure 2 is a side view of the unmanned vehicle 3 according to the invention, which is provided with a chassis 5 with wheels 6. The wheels 6 are driven by a (non-shown) drive unit. On the chassis 5 there is disposed a rotatable upper part 7 on which the detection means 4 are mounted. In the present embodiment the detection means 4 comprise a radar 8 and a camera 9. The unmanned vehicle 3 is further provided with a transmitter unit including a transmitting element 10. By means of the transmitter unit the supervisor can be alarmed when an animal is ill and/or displays abnormal behaviour. On the rotatable upper part 7 there are further disposed disinfecting means 11 for disinfecting at least a part of the stable and/or at least a part of an animal. In the present embodiment the disinfecting means 11 comprise a sprayer 12 which is disposed on a telescopic carrier 13.

On the rotatable upper part 7 there are further disposed driving means 14 for driving animals. The driving means 14 are fitted to the upper side of the camera 9. The driving means 14 are connected to an electric shock device which is capable of emitting a pulse.

In the present embodiment the camera 9 is used to guide the unmanned vehicle through the stable and/or the meadow.

## Claims

1. An unmanned vehicle to be used in a stable (1), such as e.g. a cowshed, or in a meadow, said unmanned vehicle (3) being provided with detection means (4) for determining the health and/or the behaviour of animals, **characterized in that** the unmanned vehicle (3) comprises disinfecting means (11) for disinfecting at least a part of an animal.

2. An unmanned vehicle as claimed in claim 1, **characterized in that** the disinfecting means (11) are disposed on a telescopic carrier (13).

3. An unmanned vehicle as claimed in claim 1 or 2, **characterized in that** the detection means (4) comprise an animal identification system.

4. An unmanned vehicle as claimed in claim 3, **characterized in that** the animal identification system comprises a transmitter and a receiver.

5. An unmanned vehicle as claimed in claim 3, **characterized in that** the animal identification system comprises a radar (8) as well as reflectors reacting to the radar.

6. An unmanned vehicle as claimed in claim 3, **characterized in that** the detection means comprise a camera (9).

7. An unmanned vehicle as claimed in claim 6, **characterized in that** the camera (9) is constituted by an infrared camera.

8. An unmanned vehicle as claimed in any one of the proceding claims, **characterised in that** the detection means (4) are disposed on a telescopic carrier.

9. An unmanned vehicle as claimed in any one of the preceding claims, **characterized in that** the unmanned vehicle (3) is provided with driving means (14) for driving animals.

10. An unmanned vehicle as claimed in claim 9, **characterized in that** the driving means (14) comprise an electric shock device.

11. An unmanned vehicle as claimed in any one of the preceding claims, **characterized in that** the unmanned vehicle (3) is provided with a manure slide for removing manure which is lying on a floor.

12. An unmanned vehicle as claimed in any one of the preceding claims, **characterized in that** the unmanned vehicle (3) comprises alarm means for alarming a supervisor when an animal is ill or displays abnormal behaviour.

13. An unmanned vehicle as claimed in any one of the preceding claims, **characterized in that** the unmanned vehicle (3) is provided with navigation means for guiding the unmanned vehicle through the stable or the meadow.

14. An unmanned vehicle as claimed in any one of the preceding claims, **characterized in that** the unmanned vehicle (3) comprises a rotatable upper part (7) on which the detection means (4) are disposed.

## Patentansprüche

1. Unbemanntes Fahrzeug zur Verwendung in einem Stall (1), wie z. B. einem Kuhstall, oder auf einer Wiese, wobei das unbemannte Fahrzeug (3) mit einer Überwachungsvorrichtung (4) zum Überwachen der Gesundheit und/oder des Verhaltens von Tieren versehen ist,
**dadurch gekennzeichnet, daß** das unbemannte Fahrzeug (3) eine Desinfektionsvorrichtung (11) zum Desinfizieren zumindest eines Teiles eines Tieres umfaßt.

2. Unbemanntes Fahrzeug nach Anspruch 1,
**dadurch gekennzeichnet, daß** die Desinfektionsvorrichtung (11) an einem Teleskopträger (13) angeordnet ist.

3. Unbemanntes Fahrzeug nach Anspruch 1 oder 2,
**dadurch gekennzeichnet, daß** die Überwachungsvorrichtung (4) ein Tieridentifikationssystem umfaßt.

4. Unbemanntes Fahrzeug nach Anspruch 3,
**dadurch gekennzeichnet, daß** das Tieridentifikationssystem einen Sender und einen Empfänger umfaßt.

5. Unbemanntes Fahrzeug nach Anspruch 3,
**dadurch gekennzeichnet, daß** das Tieridentifikationssystem ein Radar (8) sowie auf das Radar reagierende Reflektoren umfaßt.

6. Unbemanntes Fahrzeug nach Anspruch 3,
**dadurch gekennzeichnet, daß** die Überwachungsvorrichtung eine Kamera (9) umfaßt.

7. Unbemanntes Fahrzeug nach Anspruch 6,
**dadurch gekennzeichnet, daß** die Kamera (9) durch eine Infrarot-Kamera gebildet ist.

8. Unbemanntes Fahrzeug nach einem der vorhergehenden Ansprüche,
**dadurch gekennzeichnet, daß** die Überwachungsvorrichtung (4) an einem Teleskopträger angeordnet ist.

9. Unbemanntes Fahrzeug nach einem der vorhergehenden Ansprüche,
**dadurch gekennzeichnet, daß** das unbemannte Fahrzeug (3) mit einer Treibvorrichtung (14) zum Treiben von Tieren versehen ist.

10. Unbemanntes Fahrzeug nach Anspruch 9,
**dadurch gekennzeichnet, daß** die Treibvorrichtung (14) eine Elektroschockvorrichtung umfaßt.

11. Unbemanntes Fahrzeug nach einem der vorhergehenden Ansprüche,
**dadurch gekennzeichnet, daß** das unbemannte Fahrzeug (3) mit einem Mistschieber zum Entfernen von auf einem Boden liegendem Mist versehen ist.

12. Unbemanntes Fahrzeug nach einem der vorhergehenden Ansprüche,
**dadurch gekennzeichnet, daß** das unbemannte Fahrzeug (3) eine Alarmvorrichtung zum Alarmieren eines Aufsehers umfaßt, wenn ein Tier krank ist oder ein ungewöhnliches Verhalten zeigt.

13. Unbemanntes Fahrzeug nach einem der vorhergehenden Ansprüche,
**dadurch gekennzeichnet, daß** das unbemannte Fahrzeug (3) mit einer Navigationsvorrichtung zum Leiten des unbemannten Fahrzeuges durch den Stall oder über die Wiese versehen ist.

14. Unbemanntes Fahrzeug nach einem der vorhergehenden Ansprüche,
**dadurch gekennzeichnet, daß** das unbemannte Fahrzeug (3) einen drehbaren oberen Teil (7) umfaßt, auf dem die Überwachungsvorrichtung (4) angeordnet ist.

## Revendications

1. Véhicule téléguidé destiné à être utilisé dans une étable (1), telle que par exemple une étable pour les vaches, ou dans un pré, ledit véhicule téléguidé (3) étant muni de moyens de détection (4) pour déterminer l'état de santé et / ou le comportement des animaux, **caractérisé en ce que** le véhicule téléguidé (3) comprend des moyens de désinfection (11) pour désinfecter au moins une partie d'un animal.

2. Véhicule téléguidé selon la revendication 1, **caractérisé en ce que** les moyens de désinfection (11) sont placés sur un support télescopique (13).

3. Véhicule téléguidé selon la revendication 1 ou 2, **caractérisé en ce que** les moyens de détection (4) comprennent un système d'identification des animaux.

4. Véhicule téléguidé selon la revendication 3, **caractérisé en ce que** le système d'identification des animaux comprend un transmetteur et un récepteur.

5. Véhicule téléguidé selon la revendication 3, **caractérisé en ce que** le système d'identification des animaux comprend un radar (8) ainsi que des réflecteurs réagissant au radar.

6. Véhicule téléguidé selon la revendication 3, **caractérisé en ce que** les moyens de détection comprennent une caméra (9).

7. Véhicule téléguidé la revendication 6, **caractérisé en ce que** la caméra (9) est constituée d'une caméra infrarouge.

8. Véhicule téléguidé selon l'une quelconque des revendications précédentes, **caractérisé en ce que** les moyens de détection (4) sont placés sur un support télescopique.

9. Véhicule téléguidé selon l'une quelconque des revendications précédentes, **caractérisé en ce que** le véhicule téléguidé (3) est muni de moyens de conduite (14) pour conduire les animaux.

10. Véhicule téléguidé selon la revendication 9, **caractérisé en ce que** les moyens de conduite (14) comprennent un dispositif émettant des chocs électriques.

11. Véhicule téléguidé selon l'une quelconque des revendications précédentes, **caractérisé en ce que** le véhicule téléguidé (3) est muni d'une glissière à fumier pour enlever le fumier qui est déposé sur un sol.

12. Véhicule téléguidé selon l'une quelconque des revendications précédentes, **caractérisé en ce que** le véhicule téléguidé (3) comprend un moyen d'alerte pour alerter un superviseur lorsqu'un animal est malade ou qu'il a un comportement anormal.

13. Véhicule téléguidé selon l'une quelconque des revendications précédentes, **caractérisé en ce que** le véhicule téléguidé (3) est muni de moyens de navigation pour guider le véhicule téléguidé à travers l'étable ou le pré.

14. Véhicule téléguidé selon l'une quelconque des revendications précédentes, **caractérisé en ce que** le véhicule téléguidé (3) comprend une partie supérieure rotative (7) sur laquelle les moyens de détection (4) sont placés.
